(19) Europäisches Patentamt
European Patent Office
Office européen des brevets

(11) Publication number : **0 530 992 A2**

# (12) EUROPEAN PATENT APPLICATION

(21) Application number : **92307367.0**

(51) Int. Cl.[5] : **C07K 5/06,** C12P 21/02

(22) Date of filing : **12.08.92**

(30) Priority : **13.08.91 US 744315**

(43) Date of publication of application :
**10.03.93 Bulletin 93/10**

(84) Designated Contracting States :
**AT BE CH DE DK ES FR GB GR IE IT LI LU MC NL PT SE**

(71) Applicant : **BEND RESEARCH, INC.**
**64550 Research Road**
**Bend Oregon, 97701 (US)**

(72) Inventor : **van Eikeren, Paul**
**1922 N.W. Seventh Street**
**Bend, Oregon 97701 (US)**
Inventor : **Blair, West J.**
**63278 Whitewing Court**
**Bend, Oregon 97701 (US)**

(74) Representative : **Skerrett, John Norton Haigh et al**
**H.N. & W.S. SKERRETT Charles House 148/9 Great Charles Street**
**Birmingham B3 3HT (GB)**

(54) **Enzymatic deprotection of peptides.**

(57) A selective enzymatic hydrolysis of peptide acyl donor components useful in renin inhibitor and other tripeptide syntheses is disclosed. Enzyme preparations useful in thehydrolysis are of sulfhydryl proteases from plant sources such as papaya, pineapple and fig.

EP 0 530 992 A2

Background of the Invention

Renin inhibitors of the general structure

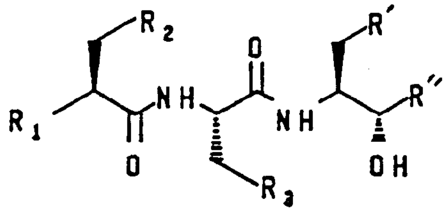

(where R′ and R″ are various substituents and $R_1$, $R_2$ and $R_3$ are as defined below) are a known class of antihypertensive agents that are the subject of increasing interest as cardiovascular drugs. See Greenlee, "Renin Inhibitors," 4 Pharm. Res. 364 (1987). Because of this, there has been considerable interest in the development of efficient methods of synthesizing certain classes of peptides exhibiting renin-inhibition properties, as well as intermediates thereof.

Such renin inhibitors are conventionally prepared by the reaction of amino acid or peptide derivatives (peptide acyl donors) of the general structure I

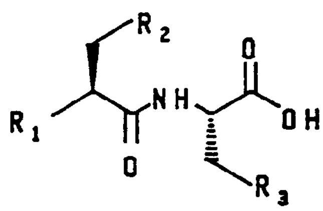

I

with β-hydroxy amine derivatives of the general structure

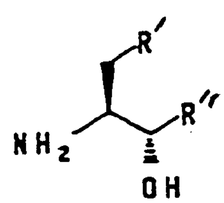

in the presence of chemical coupling reagents to form a peptide bond. See, for example, European patent applications EP312157A2, EP309841A2, and EP310070A2. In copending application Serial No. , filed on the same date herewith, there is disclosed an enzymatic synthesis of such renin inhibitors. In both the conventional and enzymatic syntheses, peptide acyl donors of the general structure I are important intermediates.

The substituents of structure I are defined as follows:

$R_1$ is ROCONH-, ROCOO-, $ROCOCH_2$-, RNHCONH-, RNHCOO-, $RNHCOCH_2$-, $RSO_2NH$-, or $RSO_2CH_2$-;

$R_2$ is cycloalkyl, aryl or aralkyl;

$R_3$ is hydrogen, alkyl, cycloalkyl, 4-imidazoyl, -OH, -SR, $-(CH_2)_n$-SR, or $-(CH_2)_n-NH_2$ where n is an integer from 1 to 6;

"alkyl" is substituted or unsubstituted straight or branched chain carbon groups of 1 to 20 carbon atoms, preferably 1 to 6 carbon atoms, which may contain heteroatoms;

"cycloalkyl" is a ring structure containing 3 to 7 carbon atoms, which may contain bridged structures or heteroatoms in the ring where the heteroatom is selected from oxygen, nitrogen and sulfur, or substituted cycloalkyl containing at least one substituent that may contain an atom selected from nitrogen, halogen, carbon, oxygen, phosporous and sulfur;

"aryl" is monocyclic, bicyclic or hetereocyclic aromatic groups containing 6 to 10 carbon atoms where

the heteroatom is selected from oxygen, nitrogen and sulfur or substituted aryl containing at least one substituent that many contain an atom selected from nitrogen, halogen, carbon, oxygen, phosphorous and sulfur; and

"aralkyl" is an alkyl group bonded to an aryl group.

Structure II, shown below, is merely the ester form of structure I, and has the same substituents as structure I except for the "R" substituent, which is alkyl, cycloalkyl, aryl or aralkyl, as defined above.

Preparation of the acid form of the general structure I requires two steps. In the first step, shown below, the $R_1$,$R_2$-containing-acyl component A is coupled to the $R_3$-containing amine component B to form the ester form II. In order to prevent the $R_3$-containing component B from coupling to itself, its carboxyl group must be protected, typically by use of the corresponding R substituent-containing ester.

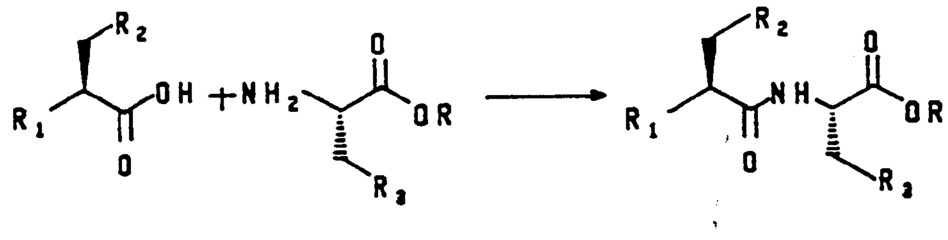

A B II

In the second step, shown below, the carboxyl group in the ester form of the coupling product II must be deprotected to produce the free acid form I.

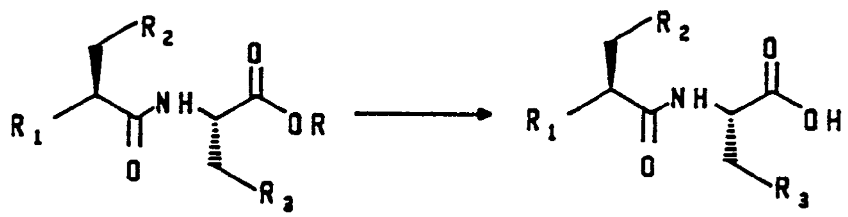

II I

Such deprotection involves the hydrolytic cleavage of the carboxyl-blocking group R in structure II above. However, such cleavage by conventional non-enzymatic means, such as with the use of alkaline earth hydroxides in water or water/organic solvent mixtures, often leads to hydrolysis of the amide linkage or to racemization of the peptide, rendering the intermediate useless. See Kemp, "Racemization in Peptide Synthesis" 1 The Peptides 315 (Gross et al. eds. 1979). Roger et al., in 101 JACS 3394 (1979), disclose an enzymatic cleavage of a carboxyl-blocking group from a peptide with the exopeptidase carboxypeptidase Y that is not suitable in the present invention since its use at substantially neutral pH leads to significant cleavage of the dipeptides of concern. Although Hermann et al., in Peptides 1982, pages 399-402 (1983) disclose the use of the endopeptidase thermitase from the microorganism Thermoactinomyces vulgaris as a catalyst to hydrolytically deprotect the terminal ester group of peptides, such enzymes were subsequently shown to cleave the peptide bond. See Dudek and Hermann, 520 J. Chrom. 333 (1990). Brubacher and Zaher, in 57 Can. J. Biochem 1064 (1979), disclose a papain-catalyzed cleavage of a series of N-protected dipeptide esters and showed that the enzyme catalyzes the cleavage of the hydrolysis of the ester linkage only. However, there is no disclosure of hydrolysis of dipeptide esters of the structure II and no disclosure of any stereoselectivity of the hydrolysis. Schechter and Berger, in 27 Biochem Biophys Research Communications 157 (1967), disclose studies on the action of papain on the hydrolysis of diastereomeric alanine oligopeptides ranging in length from dipeptide to hexapeptide. On the basis of cleavage patterns, the authors inferred that the enzyme contained seven subsites (four subsites above the catalytic site (designated $S_1$ through $S_4$) and three subsites below the catalytic site (designated $S_1$, through $S_3$,)). It was further theorized that the enzyme preferentially cleaved alanine oligopeptide epimers in which the configuration of alanine in subsite $S_1$ and $S_2$ was of the L-configuration. However, reaction had to be carried out under acidic conditions (pH 3.7) and there was no disclosure of hydrolysis of dipeptide esters of the structure II. Moreover, there was no recognition that such hydrolysis reactions can be used as a practical method to prepare N-protected dipeptide acids substantially free of diastereomeric impurities.

Summary of the Invention

It has been found that the sulfhydryl proteases papain, chymopapain, ficin and bromelain may be used to selectively cleave terminal carboxyl-blocking groups from peptides of the general structure II to prepare dipeptide acids of the structure I that are substantially free of diastereomeric impurities.

Detailed Description of the Invention

According to the present invention there is provided an enzymatic method of preparing a peptide acid of the structure I

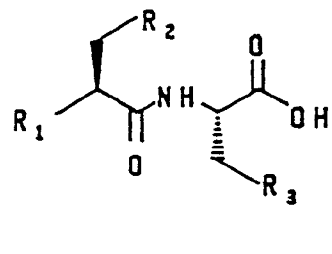

I

by selectively cleaving the carboxyl terminal ester in a peptide of the structure II

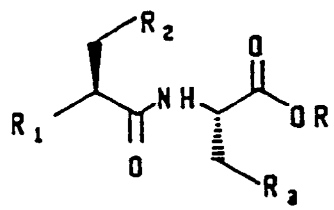

II

comprising reacting said peptide ester at a substantially neutral pH in the presence of a sulfhydryl protease preparation selected from papain, chymopapain, ficin and bromelain where R, $R_1$, $R_2$ and $R_3$ are as defined above. When so prepared, the peptide acid is substantially free of diastereomeric impurities, i.e., the isolated peptide acid of structure I contains less than 0.5% of said structure wherein the carbon bearing the $-CH_2R_3$ group has epimerized. The form in which such protease preparations may be used includes a powder, a solution, immobilized on a support, an enzyme-containing cellular extract, and enzyme-containing cells. All of these forms may be dissolved or suspended in water, an organic solvent, or a mixture of the two. When in the form of enzyme-containing cells, the cells may be whole or fragmented.

Immobilization of the protease preparation on an inert support such as bleached or unbleached or calcined diatomaceous earth, polydextran, polyacrylamide, polystyrene, polyacrylic acid ester, silica gel, or porous glass beads is also advantageous in conducting the deprotection process of the present invention. Preferred commercially available forms of calcined diatomaceous earth are those made and sold by Johns Manville of Denver, Colorado as the "Celite" series. Preferred commercially available forms of polyacrylic acid ester are those made and sold under the names "XAD-7" and "XAD-8" by Rohm & Haas of Philadelphia, Pennsylvania. Preferred forms of polystyrene are "XAD-2" and "XAD-4" by Rohm & Haas. A preferred commercially available form of polydextran is made and sold under the name "Sephadex" by the Swedish company Pharmacia. Preferred commercially available forms of polyacrylamide are those made and sold under the "Biogel P" series by Bio-Rad Laboratories of Richmond, California. Water content of such immobilized enzymatic catalysts should be from 0.5 to 15 wt%, preferably 10 wt%. Enzyme loading, based upon weight of the support, should be from 2.0 to 20 wt%, preferably 5.0 wt%. Cystamine, cysteine, beta-mercaptoethanol, hydrogen sulfide, and dithiothreitol may be added to the immobilized enzyme preparation or to the solution or suspension as an adjuvant in an amount from 1.0 to 5.0 wt%, preferably 2.0 wt%.

By "substantially neutral pH" is meant pH in the range of approximately 6.5 to 7.5.

Unreacted ester, which would contain the D-ester diastereomeric impurities, are readily removed at the

end of the hydrolysis by adjusting the pH of the aqueous layer at a value above the pKa of the dipeptide acid to form the dipeptide carboxylate anion, which is highly soluble in water. Unreacted ester can then be removed by extraction with an organic solvent like ethyl acetate, chloroform, or methylene chloride. The desired dipeptide acid can be isolated by adjusting the pH of the aqueous layer below the pKa, which causes the dipeptide acid to precipitate, and collecting the precipitate by filtration.

In all of the Examples herein, reference is made to various specific forms of the general structures I and II, set forth in Table 1, wherein all $R_2$ and $R_3$ substituents are of the S-configuration unless designated (R). The Examples are summarized in Table 2.

Table 1

| Structure No. | $R_1$ | $R_2$ | $R_3$ | R | Abbreviations |
|---|---|---|---|---|---|
| Ia | $(CH_3)_3COCONH-$ | -phenyl | -H | -H | Boc-Phe-Ala-OH |
| Ib | do | -phenyl (R) | do | do | Boc-D-Phe-Ala-OH |
| Ic | do | -phenyl | -H (R) | do | Boc-Phe-D-Ala-OH |
| Id | do | do | $-SCH_2C_6H_5$ | do | Boc-Phe-(SBzl)Cys-OH |
| Ie | do | $-C_6H_4OCH_3$ | $-SCH_3$ | do | Boc-(OMe)Tyr-(SMe)Cys-OH |
| If | do | -phenyl | -imidazoyl | do | Boc-Phe-His-OH |
| Ig | do | do | $-CH(CH_3)_2$ | do | Boc-Phe-Leu-OH |
| Ih | do | do | $-CH_2SCH_3$ | do | Boc-Phe-Met-OH |
| Ii | do | do | $-CH_2SCH_3$ (R) | do | Boc-Phe-D-Met-OH |
| Ij | do | do | -phenyl | do | Boc-Phe-Phe-OH |
| Ik | do | do | -phenyl (R) | do | Boc-Phe-D-Phe-OH |
| Il | N-morpholino-CONH- | do | $-SCH_2C_6H_5$ | do | Mor-Phe-(SBzl)Cys-OH |
| Im | do | do | -imidazoyl | do | Mor-Phe-His-OH |
| In | do | do | -imidazoyl (R) | do | Mor-Phe-D-His-OH |
| Io | do | do | $-CH(CH_3)_2$ | do | Mor-Phe-Leu-OH |
| Ip | do | do | $-SCH_3$ | do | Mor-Phe-(SMe)Cys-OH |
| Iq | do | do | $-CH_2SCH_3$ | do | Mor-Phe-Met-OH |
| Ir | $CH_3CONH-$ | do | -H | do | Ac-Phe-Ala-OH |
| IIa | $(CH_3)_3COCONH-$ | -phenyl | -H | $-CH_3$ | Boc-Phe-Ala-OMe |
| IIb | do | -phenyl (R) | -H | do | Boc-D-Phe-Ala-OMe |
| IIc | do | -phenyl | -H (R) | do | Boc-Phe-D-Ala-OMe |
| IId | do | do | $-SCH_2C_6H_5$ | do | Boc-Phe-(SBzl)Cys-OMe |
| IIe | do | $-C_6H_4OCH_3$ | $-SCH_3$ | do | Boc-(OMe)Tyr-(SMe)Cys-OMe |
| IIf | do | -phenyl | -imidazoyl | do | Boc-Phe-His-OMe |
| IIg | do | do | $-CH(CH_3)_2$ | do | Boc-Phe-Leu-OMe |
| IIh | do | do | $-CH_2SCH_3$ | do | Boc-Phe-Met-OMe |
| IIi | do | do | $-CH_2SCH_3$ (R) | do | Boc-Phe-D-Met-OMe |
| IIj | do | do | -phenyl | do | Boc-Phe-Phe-OMe |
| IIk | do | do | -phenyl (R) | do | Boc-Phe-D-Phe-OMe |
| IIl | do | do | $-SCH_2C_6H_5$ | do | Mor-Phe-(SBzl)Cys-OMe |
| IIm | N-morpholino-CONH- | do | -imidazoyl | do | Mor-Phe-His-OMe |
| IIn | do | do | -imidazoyl (R) | do | Mor-Phe-D-His-OMe |
| IIo | do | do | $-CH(CH_3)_2$ | do | Mor-Phe-Leu-OMe |
| IIp | do | do | $-SCH_3$ | do | Mor-Phe-(SMe)Cys-OMe |
| IIq | do | do | $-CH_2SCH_3$ | do | Mor-Phe-Met-OMe |
| IIr | $CH_3CONH-$ | do | -H | do | Ac-Phe-Ala-OMe |

Table 2

| Example No. | Structure No. | $R_1$ | $R_2$ | $R_3$ | R | Abbreviations |
|---|---|---|---|---|---|---|
| 1-9,18,21-24, | IIp | N-morpholino-CONH- | -phenyl | $-SCH_3$ | $-CH_3$ | Mor-Phe-(SMe)Cys-OMe |
| 36,39-41 | Ip | do | do | do | -H | Mor-Phe-(SMe)Cys-OH |
| 10,25,42 | IIa | $(CH_3)_3COCONH-$ | do | -H | $-CH_3$ | Boc-Phe-Ala-OMe |
| | Ia | do | do | -H | -H | Boc-Phe-Ala-OH |
| 11,27 | IId | do | do | $-SCH_2C_6H_5$ | $-CH_3$ | Boc-Phe-(SBzl)Cys-OMe |
| | Id | do | do | do | -H | Boc-Phe-(SBzl)Cys-OH |
| 12,28 | IIe | do | $-C_6H_4OCH_3$ | $-SCH_3$ | $-CH_3$ | Boc-(OMe)Tyr-(SMe)Cys-OMe |
| | Ie | do | do | do | -H | Boc-(OMe)Tyr-(SMe)Cys-OH |
| 13,29 | IIf | do | phenyl | -imidazoyl | $-CH_3$ | Boc-Phe-His-OMe |
| | If | do | do | do | -H | Boc-Phe-His-OH |
| 14,30 | IIg | do | do | $-CH(CH_3)_2$ | $-CH_3$ | Boc-Phe-Leu-OMe |
| | Ig | do | do | do | -H | Boc-Phe-Leu-OH |
| 15,31,44 | IIh | do | do | $-CH_2SCH_3$ | $-CH_3$ | Boc-Phe-Met-OMe |
| | Ih | do | do | do | -H | Boc-Phe-Met-OH |
| 16,32,46 | IIj | do | do | -phenyl | $-CH_3$ | Boc-Phe-Phe-OME |
| | Ij | do | do | do | -H | Boc-Phe-Phe-OH |
| 17,33 | IIl | N-morpholino-CONH- | do | $-SCH_2C_6H_5$ | $-CH_3$ | Mor-Phe-(SBzl)Cys-OMe |
| | Il | do | do | do | -H | Mor-Phe-(SBzl)Cys-OH |
| 19,34,48 | IIm | do | do | -imidazoyl | $-CH_3$ | Mor-Phe-His-OMe |
| | Im | do | do | do | -H | Mor-Phe-His-OH |
| 20,35 | IIo | do | do | $-CH(CH_3)_2$ | $-CH_3$ | Mor-Phe-Leu-OMe |
| | Io | do | do | do | -H | Mor-Phe-Leu-OH |
| 26 | IIb | $(CH_3)_3COCONH-$ | -phenyl (R) | -H | $-CH_3$ | Boc-D-Phe-Ala-OMe |
| | Ib | do | do | do | -H | Boc-D-Phe-Ala-OH |
| 37 | IIq | N-morpholino-CONH- | -phenyl | $CH_2SCH_3$ | $-CH_3$ | Mor-Phe-Met-OMe |
| | Iq | do | do | do | -H | Mor-Phe-Met-OH |

Table 2 (cont.)

| Example No. | Structure No. | $R_1$ | $R_2$ | $R_3$ | R | Abbreviations |
|---|---|---|---|---|---|---|
| 38 | IIr | $CH_3CONH-$ | -phenyl | -H | $-CH_3$ | Ac-Phe-Ala-OMe |
| | Ir | do | do | do | -H | Ac-Phe-Ala-OH |
| 43 | IIc | $(CH_3)_3COCONH-$ | do | -H (R) | $-CH_3$ | Boc-Phe-D-Ala-OMe |
| | Ic | do | do | do | -H | Boc-Phe-D-Ala-OH |
| 45 | IIi | do | do | $-CH_2SCH_3$ (R) | $-CH_3$ | Boc-Phe-D-Met-OMe |
| | Ii | do | do | do | -H | Boc-Phe-D-Met-OH |
| 47 | IIk | do | do | -phenyl (R) | $-CH_3$ | Boc-Phe-D-Phe-OMe |
| | Ik | do | do | do | -H | Boc-PHe-D-Phe-OH |
| 49 | IIn | N-morpholino-CONH- | -phenyl | -imidazoyl (R) | $-CH_3$ | Mor-Phe-D-His-OMe |
| | In | do | do | do | -H | Mor-Phe-D-His-OH |

Example 1

This Example and Examples 2-4 demonstrate that the deprotection process of the present invention can be carried out in a single phase reaction. One gram of an esterified peptide having the structure IIp was dissolved in 10 ml of a mixture of 0.1M phosphate buffer at pH 7.5 and 30 vol% acetonitrile, and 100 mg of papain preparation from papaya and 50 mg of L-cysteine were added. The mixture was stirred at 50°C and the reaction progress was monitored by assaying for the hydrolyzed peptide Ip using High Performance Liquid Chromatography (HPLC). The pH of the reaction was maintained between 6.5 and 7.5 by the addition of 0.1 N NaOH. In the course of 10 hours, 100% of IIp was hydrolyzed to Ip, detected by HPLC. The product was isolated as follows. The organic solvent was removed by evaporation under vacuum. The pH was adjusted to 7 by the addition of 0.1 N NaOH, and the reaction washed 3 times with ethyl acetate. The pH of the aqueous phase was adjusted to 2 by the addition of 0.2 M HCl and the aqueous phase was extracted 3 times with ethyl acetate. The ethyl acetate extracts were combined and concentrated under vacuum until the product crystallized. Solid was collected by filtration and dried to give 0.7 g of Ip, an 83% yield.

### Example 2

Example 1 was repeated in 0.1M phosphate buffer at pH 7.5 in 30 vol% dioxane. The reaction was monitored with HPLC and was complete in 10 hours, with substantially the same yield.

### Example 3

Example 1 was repeated in 0.1M phosphate buffer at pH 7.5 in 30 vol% acetone. The reaction was monitored with HPLC and was complete in 10 hours, with substantially the same yield.

### Example 4

Example 1 was repeated in 0.1M phosphate buffer at pH 7.5 in 30 vol% tetrahydrofuran. The reaction was monitored with HPLC and was complete in 10 hours, with substantially the same yield.

### Example 5

This Example and Examples 6-8 demonstrate that the process of the present invention can be carried out in a two-phase reaction system. The advantages of a two-phase reaction are (1) higher concentrations of dipeptide can be deprotected, and (2) isolation and purification of the deprotected dipeptide is simpler than in the case of a single phase reaction. Four ml of 250 mM of the peptide ester having the structure IIp in ethyl acetate (EtOAc) was added to 1 ml of 0.1M phosphate buffer at pH 7.5, containing the same catalyst preparation and adjuvant in the same amounts as in Example 1. The mixture was stirred at 50°C and the organic phase monitored for the presence of Ip by HPLC. After 8 hours, no starting ester (IIp) could be detected. The pH of the aqueous phase was dropped to 2 by the addition of 0.2M HCl and the mixture vigorously stirred for 10 minutes. The EtOAc phase was separated, dried over $MgSO_4$, and concentrated under vacuum, yielding 280 mg crystals of Ip, a 70% yield.

### Example 6

Example 5 was repeated with a ratio of organic solvent to buffer of 1:4 with substantially the same results.

### Example 7

Example 5 was repeated with a ratio of organic solvent to buffer of 20:1 with substantially the same results.

### Example 8

This Example illustrates that the process of the present invention can be performed by an immobilized enzyme preparation in a water-immiscible solvent, whereby isolation and purification of the deprotected dipeptide is simplified, and the catalyst is removed by filtration. Five ml of 250 mM of the ester IIp in water-saturated EtOAc was added to 1.25 g of 5 wt% papain from papaya on Celite (water content 10 wt%). After 8 hours, no starting ester could be detected. The Celite was filtered, washed with EtOAc, the combined EtOAc washes were dried with $MgSO_4$, and then concentrated under vacuum to yield 0.3 g Ip, a 62% yield.

### Example 9

A solution of 1 ml of 250 mM IIp in acetonitrile containing 10 wt% water was added to 0.25 g of the same supported catalyst used in Example 7. After 8 hours, the ester was 97.7% hydrolyzed, as detected by HPLC.

### Examples 10-20

These Examples illustrate that a substantial number of different peptide structures are<amenable to deprotection by the process of the present invention. Peptide structures hydrolyzed are shown in Table 3. Dipeptide esters (250 mM) were dissolved in water-saturated EtOAc in the presence of substantially the same catalyst preparation used in Example 7, the water content varying between 10 and 15 wt%, and the mixture was stirred at 50°C. Specific initial rates were calculated by initial slopes of plots of reaction progress, as measured by HPLC, and quantitative hydrolysis took place in each case.

Table 3

| Ex. No. | Dipeptide Ester | Dipeptide Acid | Normalized Initial Rate (mM/hr/wt% enz) | Time To 95% Conversion (hrs) |
|---|---|---|---|---|
| 10 | IIa | Ia | 380 | 3 |
| 11 | IId | Id | 300 | 1.5 |
| 12 | IIe | Ie | 50 | 9 |
| 13 | IIf | If | 170 | 4.5 |
| 14 | IIg | Ig | 20 | 10 |
| 15 | IIh | Ih | 270 | 2 |
| 16 | IIj | Ij | 48 | 8 |
| 17 | IIl | Il | 300 | 3 |
| 18 | IIp | Ip | 190 | 3.5 |
| 19 | IIm | Im | 110 | 6 |
| 20 | IIo | Io | 24 | 8.5 |

Examples 21-24

These Examples demonstrate the usefulness of various other sulfhydryl endopeptidase preparations useful as catalysts in the present invention. Enzymes at 5 wt% loading were immobilized on Celite 649, with 2 wt% cysteine adjuvant. A solution of 250 mM of IIp in water saturated EtOAc was combined with 250 mg/ml of the immobilized enzyme, resulting in quantitative hydrolysis of the dipeptide ester in each case. Other results are shown in Table 4.

Table 4

| Ex. No. | Protease | Normalized Initial Rate (mM/hr/wt% enz) | Time to 95% Conversion (hrs) |
|---|---|---|---|
| 21 | chymopapain | 300 | 2 |
| 22 | ficin | 260 | 2 |
| 23 | papain | 190 | 3.5 |
| 24 | bromelain | 39 | 10 |

Examples 25-47

These Examples demonstrate the regio-selectivity of the process of the present invention. Regioselectivity is the capability of an enzyme to hydrolyze the group -OR from structure II, giving structure I, without cleaving the internal peptide bond between the amino acid bearing the substituent $R_2$ and the amino acid bearing the substituent $R_3$. In the case of IIp, a fully regioselective process will produce only compound Ip, without the production of any peptide cleavage products.

To demonstrate such regioselectivity, a solution of 250 mM peptide in water-saturated EtOAc was added to 5 wt% enzyme preparation on Celite 648 and 2 wt% cysteine adjuvant. The sensitivity of the analysis was such that it was capable of detecting 0.01% of any other cleavage products. All deprotection reactions were analyzed by HPLC for the presence of the cleavage products Mor-(or Boc)-Phe-OH as an indicator of internal cleavage of the dipeptide ester. The results of these analyses are shown in Table 5. Regioselectivity was defined as the ratio of normalized initial rate of ester hydrolysis to the normalized initial rate of cleavage product production. When no cleavage products were detected, a rate of 0.8 μM/hr/wt% enzyme was used as an upper limit for the calculation.

Table 5

| Ex. No. | Peptide | Produce | Protease | Minimum Regio-selectivity |
|---|---|---|---|---|
| 25 | IIa | Ia | papain | 460000 |
| 26 | IIb | Ib | papain | 71000 |
| 27 | IId | Id | papain | 360000 |
| 28 | IIe | Ie | papain | 63000 |
| 29 | IIf | If | papain | 200000 |
| 30 | IIg | Ig | papain | 23000 |
| 31 | Ilh | Ih | papain | 320000 |
| 32 | IIj | Ij | papain | 57000 |
| 33 | IIl | Il | papain | 360000 |
| 34 | IIm | Im | papain | 130000 |
| 35 | IIo | Io | papain | 29000 |
| 36 | IIp | Ip | papain | 230000 |
| 37 | IIq | Iq | papain | 210000 |
| 38 | IIr | Ir | papain | 460000 |
| 39 | IIp | Ip | chymopapain | 360000 |
| 40 | IIp | Ip | ficin | 310000 |
| 41 | IIp | Ip | bromelain | 47000 |

Examples 42-45

These Examples demonstrate that the process of the present invention is highly selective for dipeptides of structure II where the amino acid residue bearing the substituent $R_3$ is of the L-configuration. Epimeric dipeptides of Boc-Phe-Ala-OMe (compounds IIa and IIc), Boc-Phe-Met-OMe (compounds IIh and IIi), Boc-Phe-Phe-OMe (compounds IIj and IIk), and Mor-Phe-His-OMe (compounds IIm and IIn) were carboxy-deprotected with 5 wt% papain preparation immobilized on Celite 648 (4 wt% water content) in water saturated EtOAc containing 250 mM dipeptide ester. This diastereoselectivity was defined as the ratio of normalized initial rates of hydrolysis of the L-L-dipeptide ester to the L-D-dipeptide ester. Dipeptides containing carboxy terminal D-amino acids were not detectably deprotected, even after reaction times in excess of 120 hrs. On the basis of sensitivity of the analysis technique, which was capable of detecting reaction rates as slow as 0.8 μM/hr/wt% enzyme after 24 hours, the minimum diastereoselectivity of the reactions was calculated. If no hydrolysis was seen, the limit value of 0.8 μM/hr/wt% enzyme was used. Results are shown in Table 6.

Table 6

| Ex. No. | Peptide | Product | Normalized Initial Rate (mM/hr/wt% enz) | Minimum Diastereo-selectivity |
|---|---|---|---|---|
| 42 | IIa<br>IIc | Ia<br>Ic | 380<br><0.0008 | 480000 |
| 43 | IIh<br>IIi | Ih<br>Ii | 270<br><0.0008 | 340000 |
| 44 | Ilj<br>IIk | Ij<br>Ik | 48<br><0.0008 | 600000 |
| 45 | IIm<br>IIn | Im<br>In | 110<br><0.0008 | 14000 |

The terms and expressions which have been employed in the foregoing specification are used therein as terms of description and not of limitation, and there is no intention in the use of such terms and expressions of excluding equivalents of the features shown and described or portions thereof, it being recognized that the scope of the invention is defined and limited only by the claims which follow.

## Claims

1. An enzymatic hydrolysis method of preparing a peptide acid of the structure

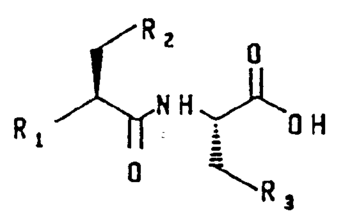

characterized by reacting a peptide of the structure

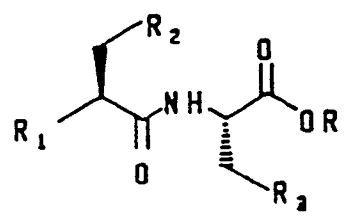

at a substantially neutral pH in the presence of a sulfhydryl protease preparation selected from papain, chymopapain, ficin, and bromalain where

R is alkyl, cycloalkyl, aryl or aralkyl;

$R_1$ is ROCONH-, ROCOO-, $ROCOCH_2$-, RNHCONH-, RNHCOO-, $RNHCOCH_2$-, $RSO_2NH$-, or $RSO_2CH_2$-;

$R_2$ is cycloalkyl, aryl or aralkyl;

$R_3$ is hydrogen, alkyl, cycloalkyl, 4-imidazoyl, -OH, -SR, $-(CH_2)_n$-SR, or $-(CH_2)_n-NH_2$ where n is an integer from 1 to 6;

alkyl is substituted or unsubstituted or heteroatom-containing straight or branched chain carbon groups of 1 to 20 carbon atoms;

cycloalkyl is a ring structure containing 3 to 7 carbon atoms, which may contain bridged structures or heteroatoms in the ring, or substituted cycloalkyl; aryl is a monocyclic, bicyclic or heterocyclic aromatic group containing 6 to 10 carbon atoms, or substituted aryl; and
aralkyl is alkyl bonded to aryl.

**2.** The method of claim 1 wherein said endopeptidase preparation is in a form selected from a powder, a solution, immobilized on a support, an enzyme-containing cellular extract, and enzyme-containing cells.

**3.** The method of claim 2 wherein the solvent for said solution of said endopeptidase preparation is selected from water, an organic solvent, and a mixture of water and an organic solvent.

**4.** The method of claim 2 wherein the solvent is 10-40 vol% aqueous acetonitrile.

**5.** The method of claim 2 wherein said enzyme-containing cells are whole cells.

**6.** The method of claim 2 wherein said enzyme-containing cells are partial cells.